# EUROPEAN PATENT SPECIFICATION

(11) **EP 2 803 377 B1**
(45) Date of publication and mention of the grant of the patent: **06.07.2022**
(21) Application number: 14162140.9
(22) Date of filing: 27.03.2014
(51) Int. Cl.: A61M 5/315

(54) **A combination of a medical gasket and a plunger for a prefilled syringe**
Eine Kombination von einer medizinischen Dichtung und einem Kolben für eine vorgefüllte Spritze
Une combination d'un joint médical et d'un piston pour une seringue préremplie

(30) Priority: 15.05.2013 JP 2013103452
(43) Date of publication of application: 19.11.2014
(73) Proprietor: SUMITOMO RUBBER INDUSTRIES, LTD., Kobe-shi, Hyogo-ken, 651-0072 (JP)
(72) Inventor: NAKANO, Hiroaki, Kobe-shi, Hyogo 651-0072 (JP); YAO, Eiji, Kobe-shi, Hyogo 651-0072 (JP); IWANO, Shinya, Kobe-shi, Hyogo 651-0072 (JP); ISHIDA, Naoyuki, Kobe-shi, Hyogo 651-0072 (JP)
(74) Representative: Manitz Finsterwald Patent- und Rechtsanwaltspartnerschaft mbB

(56) References cited:
- EP-A1- 2 407 195
- EP-A1- 2 484 393
- WO-A1-00/07648
- WO-A1-2012/134430
- GB-A- 741 604
- JP-A- 2004 121 344
- JP-A- 2009 142 508
- US-A- 3 831 601

## Description

### TECHNICAL FIELD

The present invention relates to a combination of a medical gasket and a plunger for a prefilled syringe.

### BACKGROUND ART

Syringes prefilled with a drug (prefilled syringes) are used in medical applications. Prefilled syringes eliminate the need to troublesomely transfer drugs from another container and thus are easy to use; besides, they prevent medical errors during transfer of drugs. Therefore, they are being increasingly used these days. Such prefilled syringes need to have the properties required of containers which are to be in contact with drugs for a long period of time (see JP 2012-147859 A), unlike the conventional syringes (in the case of the conventional syringes, a drug is drawn from another container, such as a vial, immediately before use).

In some prefilled syringes, the plunger rod and the gasket are previously fitted to each other. In other prefilled syringes, the plunger or plunger rod is fitted to the gasket when used, and this plunger rod is reused. In the latter case, in order to fit the gasket and the plunger rod, a female screw and a male screw may be formed in the gasket and the plunger rod, respectively, so that they can be fitted to each other. The plunger rod is then turned to fit both screw portions.

While the gasket and the plunger need to be tightly fitted to each other, there is also a demand that the force (torque) required for turning the plunger rod should be reduced to facilitate the attachment. Moreover, if the screw is excessively turned for tighter fit, then the gasket is compressed, which increases friction between the gasket and the syringe barrel. This disadvantageously results in difficulty in pushing the gasket into the barrel upon injection.

WO 00/07648 A1 and GB 741 604 A each disclose a combination in accordance with the preamble of claim 1.

Further examples of prior art gaskets and plungers can be found in EP 2407195 A1, WO 2012/134430 A1, JP 2004-121344 A and US 3 831 601 A.

### SUMMARY OF INVENTION

### TECHNICAL PROBLEM

The present invention aims to solve the above problem and reduce the clamping force required to fit a plunger rod into the gasket, while maintaining a tight fit therebetween. Claim 1 provides a combination of a medical gasket and a plunger for a prefilled syringe in accordance with the present invention.

### ADVANTAGEOUS EFFECTS OF INVENTION

The medical gasket of the present invention has a female screw portion to fit a plunger, on the side opposite to a drug contact surface. The female screw portion has roots and crests which gradually increase in diameter from the drug contact surface side toward the opposite plunger side. The present invention can therefore provide a gasket that reduces the clamping force required to fit a plunger rod into the gasket, while maintaining a tight fit therebetween.

### BRIEF DESCRIPTION OF DRAWINGS

Fig. 1 is a conceptual view illustrating the relation between a gasket and a plunger.
Fig. 2 is a cross-sectional view of an embodiment of the gasket of the present invention.
Fig. 3 is a cross-sectional view of another embodiment of the gasket of the present invention.

### DESCRIPTION OF EMBODIMENTS

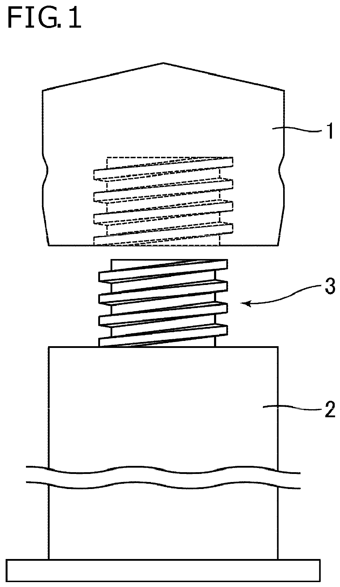

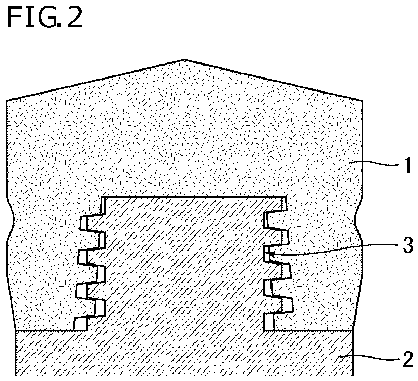

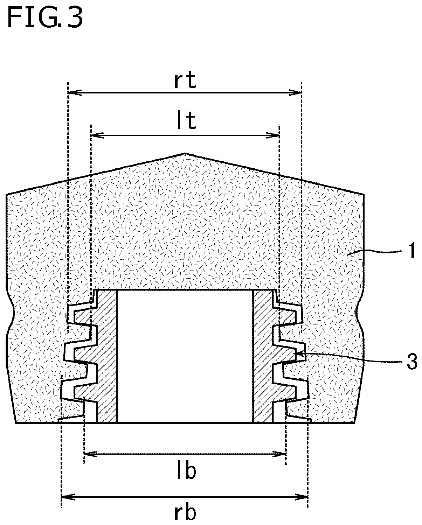

The medical gasket in accordance with the present invention has a female screw portion to fit a plunger, on the side opposite to a drug contact surface, wherein the female screw portion has roots and crests which gradually increase in diameter from the drug contact surface side toward the opposite plunger side. This structure enables the gasket to reduce the clamping force required to fit a plunger rod into the gasket, while maintaining a tight fit therebetween, when the plunger rod is inserted into the gasket as shown in Fig. 1. Figs. 2 and 3 show specific examples of the shape of the gasket and plunger of the present invention.

The root diameter r of the female screw portion of the gasket needs to be larger than the crest diameter R of the male screw portion of a plunger rod. Moreover, it is preferred that the following inequalities hold: R < rt < rb where rt represents the root diameter of the female screw portion on the liquid contact side of the gasket and rb represents the root diameter of the female screw portion on the side opposite to the liquid contact side. Here, rb is preferably at least 1.02 times rt, and more preferably at least 1.05 times rt. If rb is less than 1.02 times rt, though the gasket performs well with respect to the force required to pull out the rod, fitting torque tends to increase so that the desired effect cannot be provided. The upper limit of rb is not particularly limited, and rb is preferably at most 1.3 times rt, and more preferably at most 1.2 times rt. If rb is more than 1.3 times rt, the difference in screw root diameter between them is too large, which makes it difficult to fit the screws over the whole screw area from the liquid contact side to the opposite side, and thus tends to result in reduced pull-out force. In the present invention, the root diameter of the female screw portion gradually increases from rt to rb, but this increase is not necessarily linear. Moreover, rt is preferably at least 1.01 times R, and more preferably at least 1.03 times R. If rt is less than 1.01 times R, the roots of the female screw portion may interfere with the crests of the male screw portion so that fitting torque can be increased. The upper limit of rt is not particularly limited, and rt is preferably at most 1.3 times R, and more preferably at most 1.2 times R. If rt is more than 1.3 times R, the screws are difficult to fit to each other, which tends to result in reduced pull-out force.

In order to more tightly fit a plunger rod into the gasket, the following inequalities preferably hold: lt < lb < R where 1 represents the crest diameter of the female screw portion, more specifically, lt represents the crest diameter of the female screw portion on the liquid contact side of the gasket, and lb represents the crest diameter of the female screw portion on the side opposite to the liquid contact side. Here, lb is preferably at least 1.02 times lt, and more preferably at least 1.05 times lt. If lb is less than 1.02 times lt, though the gasket performs well with respect to the force required to pull out the rod, fitting torque tends to increase so that the desired effect cannot be provided. The upper limit of lb is not particularly limited, and is preferably at most 1.3 times lt, and more preferably at most 1.2 times It. If lb is more than 1.3 times lt, the difference in screw crest diameter between them is too large, which makes it difficult to fit the screws over the whole screw area from the liquid contact side to the opposite side, and thus tends to result in reduced pull-out force.

The crest diameter gradually increases from lt to lb, but this increase is not necessarily linear. Moreover, lb is preferably at least 0.8 times R, and more preferably at least 0.9 times R. If lb is less than 0.8 times R, the crests of the female screw portion may interfere with the root portions of the male screw portion so that fitting torque can be increased. The upper limit of lb is not particularly limited, but lb needs to be less than 1 time R.

According to the invention, both the roots and the crests of the female screw portion gradually increase in diameter from the drug contact surface side toward the opposite plunger side.

The crest diameter of the male screw portion is constant over the entire fitting area.

The female screw portion of the gasket may have any shape as long as the relations described above are satisfied. The number, the height, the shape, the roundness, and the like of the screw crests may be appropriately altered depending on those of the male screw portion of the plunger. The outer shape of the gasket, except areas that affect the female screw portion, is not particularly limited.

The plunger rod is preferably inserted under a fitting torque of 30 N·cm or lower, and more preferably 20 N·cm or lower.

The gasket base material may include any elastic material, and examples thereof include various rubber materials such as natural rubber, butyl rubber, isoprene rubber, butadiene rubber, styrene-butadiene rubber, silicone rubber, epichlorohydrin rubber, ethylene propylene rubber, and nitrile rubber; and various thermoplastic elastomers such as polyurethane-based, polyester-based, polyamide-based, olefin-based, and styrene-based thermoplastic elastomers. These elastic materials may be used alone or as a blend of two or more. Preferred among these are materials which become elastic when vulcanized. In terms of moldability, ethylene-propylene-diene rubber, butadiene rubber, and the like are preferred. In terms of gas permeation resistance, butyl rubber, chlorinated butyl rubber, brominated butyl rubber, and the like are also preferred. In the case of vulcanizable materials, additives known in the rubber industry, such as a vulcanizing agent (e.g. sulfur) and a vulcanization accelerator, may be appropriately added.

A gasket having a surface coated with a film, a so-called laminated gasket, is known. The present invention can be suitably applied to both laminated gaskets and non-coated (non-laminated) gaskets.

The film used in the laminated gasket is preferably excellent in sliding properties, i.e., the film preferably has a smaller friction coefficient than rubber. Examples of such films include ultrahigh molecular weight polyethylene and fluororesins, which have been widely used in medical applications. Preferred among these are fluororesins because they are excellent in sliding properties and have highly chemically stable surface. Any known fluororesin containing fluorine can be used, and examples thereof include PTFE, modified PTFE (copolymer of tetrafluoroethylene and a small amount of perfluoroalkoxide monomer), ethylene tetrafluoroethylene (ETFE) copolymer, and perfluoroalkylvinylether (PFA) copolymer. PTFE and modified PTFE are preferred because they are especially excellent in both sliding properties and chemical stability. ETFE is also preferred because it has good resistance to γ-radiation sterilization.

Any known silicone oil or curable silicone oil may be applied to the inner surface of the barrel or the gasket surface as long as they do not adversely affect a drug inside the barrel. Applying such oil increases sliding properties.

The gasket of the present invention may be prepared as follows. Compounding materials at predetermined ratios are kneaded in an internal mixer, an open roll mill, or the like to provide a kneaded mixture. The kneaded mixture is sheeted in a calendar or a sheeting machine to provide an unvulcanized rubber sheet. Subsequently, a sheet having a certain weight and size is cut from the unvulcanized sheet, and then the cut sheet and optionally an inert film are stacked on a mold, and molded by vacuum press to provide a molded gasket sheet.

The molding conditions are not particularly limited, and may be appropriately set. The molding temperature preferably ranges from 155°C to 200°C, and more preferably from 165°C to 180°C. The molding time is preferably 1 to 20 minutes, more preferably 3 to 15 minutes, and even more preferably 5 to 10 minutes.

Thereafter, unnecessary parts of the gasket molding are cut away and removed, and then washed, sterilized, dried, and checked for appearance. Thus, a finished gasket is obtained.

### EXAMPLES

The present invention is described in more detail with reference to, but not limited to, examples.

Materials used in the examples are listed below. Unvulcanized rubber sheet: halogenated butyl rubber Cross-linking agent:
2-di-n-butylamino-4,6-dimercapto-s-triazine (Zisnet DB, a product of Sankyo Kasei Co., Ltd.)

### Examples 1 and 2 and Comparative Example 1

Example gaskets were prepared in which the root diameter of the female screw portion gradually increased from the liquid contact side of the gasket toward the opposite side. Also, a Comparative example gasket was prepared in which the root diameter was constant. Specifically, an unvulcanized rubber sheet was placed on a mold and then molded by vacuum press at 180°C and a treatment pressure of 20 MPa for 8 minutes to perform vulcanization bonding.

Each of the gaskets had a maximum outer diameter of 20.0 mm. The root diameter and the crest diameter of the female screw portion of each gasket are shown in Table 1. The length (depth) of the screw portion was 7.0 mm, and the pitch was 17 crests per 25.4 mm.

### <Measurement of torque for fitting plunger rod>

The maximum torque required to fit the screw portion of a plunger rod (made of polypropylene resin, the crest diameter of the male screw portion: 12.6 mm, the root diameter of the male screw portion: 9.6 mm) into the screw portion of the gasket was measured with a torque wrench (BTG60CN, a product of Tohnichi Mfg. Co., Ltd.). To fit the screw portions, the screw portion was turned until the gasket surface opposite to the liquid contact side reached the top of the plunger rod. Five pairs of gaskets and plunger rods were measured, and the average value of the measurements is shown in Table 1. An average value of 20 N·cm or lower is rated as good.

### <Plunger rod pull-out test>

After the gasket was fitted to the plunger rod under the above conditions, the pull-out force required to pull the plunger rod out of the gasket was measured with an autograph (AUTOGRAPH AG-1000D, a product of SHIMADZU Corporation). The gasket and the plunger rod were each held in the chuck of the autograph and then subjected to pull-out loads at a movement speed of 500 mm/min until the plunger rod was pulled out of the gasket. The maximum load applied was determined as the pull-out force. Five pairs of gaskets and plunger rods were measured, and the average value of the measurements is shown in Table 1. An average value of 40 N or higher is rated as good.

**[Table 1]**

| | | Example | | Comparative Example |
|---|---|---|---|---|
| | | 1 | 2 | 1 |
| Diameter of female screw of gasket | Root diameter rt of female screw portion on liquid contact side (mm) | 13.2 | 13.1 | 13.1 |
| | Root diameter rb of female screw portion on opposite side (mm) | 14.5 | 13.4 | 13.1 |
| | Crest diameter lt of female screw portion on liquid contact side (mm) | 10.7 | 10.7 | 10.8 |
| | Crest diameter lb of female screw portion on opposite side (mm) | 11.6 | 11.0 | 10.8 |
| Evaluation results | Fitting torque (N·cm) | 12 | 18 | 54 |
| | Pull-out force (N) | 43.5 | 44.5 | 44.6 |

The Example gaskets, in which the root diameter of the female screw portion gradually increased from the liquid contact side of the gasket toward the opposite side, exhibited a low fitting torque and also showed good results in the pull out test. In contrast, the Comparative Example gasket, in which the root diameter of the female screw portion on the liquid contact side was the same as that on the side opposite to the liquid contact side, required a higher torque for fitting than the Example gaskets and thus was not suitable for usage, although it showed a good result in the pull out test as obtained in the Example gaskets.

### REFERENCE SIGNS LIST

1: gasket
2: plunger
3: male screw portion
rt: root diameter of female screw portion on liquid contact side of gasket
lt: crest diameter of female screw portion on liquid contact side of gasket

## Claims

1. A combination of a medical gasket (1) and a plunger (2) for a prefilled syringe, the plunger (2) comprising a male screw portion (3),
the medical gasket (1) comprising a female screw portion to fit said male screw portion (3) of the plunger (2), on a side opposite to a drug contact surface,
the female screw portion having roots and crests which gradually increase in diameter from a drug contact surface side toward an opposite plunger side, such that
a root diameter (rb) of the female screw portion on the plunger side is larger than a root diameter (rt) of the female screw portion on the drug contact surface side of the gasket (1), and
a crest diameter (lb) of the female screw portion on the plunger side is larger than a crest diameter (lt) of the female screw portion on the drug contact surface side of the gasket,
**characterized in that**
the male screw portion (3) of the plunger (2) has a constant crest diameter over the entire fitting area.

## Patentansprüche

1. Kombination aus einer medizinischen Dichtung (1) und einem Kolben (2) für eine vorgefüllte Spritze, wobei der Kolben (2) einen Außengewindeabschnitt (3) umfasst,
die medizinische Dichtung (1) einen Innengewindeabschnitt umfasst, der auf den Außengewindeabschnitt (3) des Kolbens (2) auf einer Seite gegenüber einer Arzneimittelkontaktfläche passt,
wobei der Innengewindeabschnitt Füße und Scheitel aufweist, deren Durchmesser von einer Arzneimittelkontaktflächenseite aus zu einer gegenüberliegenden Kolbenseite hin allmählich zunimmt, so dass
ein Fußdurchmesser (rb) des Innengewindeabschnitts auf der Seite des Kolbens größer ist als ein Fußdurchmesser (rt) des Innengewindeabschnitts auf der Seite der Arzneimittelkontaktfläche der Dichtung (1), und
ein Scheiteldurchmesser (lb) des Innengewindeabschnitts auf der Seite des Kolbens größer ist als ein Scheiteldurchmesser (lt) des Innengewindeabschnitts auf der Seite der Arzneimittelkontaktfläche der Dichtung,
**dadurch gekennzeichnet, dass**
der Außengewindeabschnitt (3) des Kolbens (2) über den gesamten Passbereich einen konstanten Scheiteldurchmesser aufweist.

## Revendications

1. Combinaison d'un joint médical (1) et d'un piston (2) pour une seringue préremplie, le piston (2) comprenant une portion de vissage mâle (3),
le joint médical (1) comprenant une portion de vissage femelle pour s'apparier à ladite portion de vissage mâle (3) du piston (2), sur un côté opposé à une surface de contact de médicament,
la portion de vissage femelle ayant des racines et des crêtes qui augmentent graduellement en diamètre depuis un côté surface de contact de médicament jusqu'à un côté piston opposé, de telle sorte que un diamètre de racine (rb) de la portion de vissage femelle sur le côté piston est plus grand qu'un diamètre de racine (rt) de la portion de vissage femelle sur le côté surface de contact de médicament du joint (1), et
un diamètre de crête (lb) de la portion de vissage femelle sur le côté piston est plus grand qu'un diamètre de crête (lt) de la portion de vissage femelle sur le côté surface de contact de médicament du joint,
**caractérisé en ce que**
la portion de vissage mâle (3) du piston (2) a un diamètre de crête constant sur l'ensemble de la zone d'appariement.
